# EUROPÄISCHE PATENTSCHRIFT

(11) **EP 1 839 668 B1**
(45) Veröffentlichungstag und Bekanntmachung des Hinweises auf die Patenterteilung: **24.10.2012**
(21) Anmeldenummer: 06006727.9
(22) Anmeldetag: 30.03.2006
(51) Int. Cl.: A61K 36/81, A61K 36/89, A61P 31/22

(54) **Verwendung von Nisylen zur Behandlung von Herpes labialis und Herpes genitalis**
Use of Nisyline in the treatment of Herpes labialis and Herpes genitalis
Utilisation de la Nisyline pour le traitement de l'Herpes labialis et de l'Herpes genitalis

(30) Priorität: 28.03.2006 DE 202006004962 U
(43) Veröffentlichungstag der Anmeldung: 03.10.2007
(73) Patentinhaber: Huber, Klaus, 83646 Bad Tölz (DE)
(72) Erfinder: Huber, Klaus, 83646 Bad Tölz (DE)
(74) Vertreter: Herrmann, Uwe

(56) Entgegenhaltungen:
- WO-A-03/059071

## Beschreibung

Die vorliegende Erfindung betrifft die Verwendung von Nisylen zur Herstellung eines Arzneimittels.

Nisylen ist als Arzneimittel zur Anwendung bei grippalen Infekten, Fieber, Hals- und Kopfschmerzen und Husten bekannt. Es handelt sich um ein homöopathisches Arzneimittel in Form eines Kombinationspräparates aus verschiedenen Wirkstoffen, das in Form von Tabletten oder einer Lösung zum Einnehmen erhältlich ist. Bei den Wirkstoffen handelt es sich um Aconitum Dil. D3, Gelsemium Dil. D3, lpecacuanha Dil. D3, Phosphorus Dil. D5, Bryonia Dil. D2 und Eupatorium perfoliatum.

Ebenfalls bekannt ist die Verwendung von Cepa, Euphrasia, Belladonna und Mercurius solubilis für homöopathische Arzneimittel.

Aus der WO 03/059071 A1 ist darüber hinaus die Verwendung von Allium Cepa bekannt, wobei die zu behandelnden viralen Infektionen Herpes umfassen können.

Die Aufgabe der vorliegenden Erfindung besteht darin, weitere medizinische Verwendungen einer oder mehrerer der oben angegebenen Bestandteile anzugeben.

Die Erfindung basiert auf der Erkenntnis, dass Nisylen und/oder Cepa und/oder Euphrasia und/oder Belladonna und/oder Mercurius solubilis zur Behandlung von Herpes labialis und Herpes genitalis eingesetzt werden kann/können.

Bei Herpes labialis handelt es sich um eine Viruserkrankung, die durch das Herpes-Simplex-Virus Typ 1 (HSV Typ 1) hervorgerufen wird. Dabei entstehen an den Lippen kleine, nässende Bläschen, die empfindlich sind, Schmerzen und Juckreiz verursachen und hochinfektiös sind.

Herpes genitalis wird durch den Herpes-Simplex-Virus Typ II (HSV Typ II) hervorgerufen. Auch bei Herpes genitalis bilden sich mit Flüssigkeit gefüllte Bläschen, die erhebliche Schmerzen, Neuralgien und Juckreiz verursachen und ebenfalls hochinfektiös sind.

Beide Virusarten (HSV Typ I und II) befallen Haut, Schleimhäute, Augen, das Nervensystem und selten auch innere Organe. Aufgrund der Tatsache, dass sich das Virus ständig im Körper befindet, kann es immer wieder zu Rezidiven, d.h. erneuten Ausbrüchen der Krankheit kommen. Bei den derzeit bekannten Medikamenten handelt es sich um Virustatika, die den Krankheitsverlauf verkürzen und die Beschwerden mindern.

Erfindungsgemäß wurde nun gefunden, dass ein Arzneimittel, das Nisylen enthält, zur präventiven und therapeutischen Behandlung dieser Herpesarten geeignet ist.

Die Behandlung von Patienten mit Herpes labialis sowie von Patienten mit Herpes genitalis mit dem Arzneimittel gemäß der vorliegenden Erfindung hat gezeigt, dass sich der Krankheitsverlauf in beiden Fällen erheblich verkürzt hat.

Erfindungsgemäß kann vorgesehen sein, dass das Arzneimittel einen der Wirkstoffe Cepa oder Euphrasia oder Belladonna oder Mercurius solubilis als weitere Bestandteile und die der weiteren Bestandteile als Nebenbestandteile enthält, wobei der Hauptbestandteil in größerer Menge bzw. Konzentration vorliegt als jeder der weiteren Bestandteile.

Grundsätzlich ist denkbar, dass das Arzneimittel nur Nisylen als Wirkstoff enthält.

Von der Erfindung sind beliebige Kombinationen der genannten Bestandteile umfaßt, beispielsweise ein Arzneimittel das Nisylen als Hauptbestandteil und alle anderen der genannten Bestandteile der Kombinationen von diesen als Nebenbestandteile umfaßt.

Es ist vorgesehen, dass das Arzneimittel Nisylen enthält.

In weiterer bevorzugter Ausgestaltung ist vorgesehen, dass das Arzneimittel ferner Cepa enthält. Bei Cepa handelt es sich ebenfalls um eine homöopathisch wirksame Substanz, die üblicherweise bei Schnupfen angewendet wird.

In einer weiteren Ausgestaltung der Erfindung enthält das Arzneimittel ferner Euphrasia. Euphrasia ist ebenfalls eine aus der Homöopathie bekannte Substanz, die üblicherweise bei Erkrankungen der Augen eingesetzt wird.

Weitere Bestandteile des Arzneimittels gemäß der vorliegenden Erfindung können Belladonna und / oder Mercurius solubilis darstellen. Belladonna wird derzeit als homöopathisches Medikament bei verschiedenen Formen von Entzündungen eingesetzt. Mercurius solublilis ist ein homöopathisches Medikament, das zur Behandlung unter anderem von Halsentzündung, Mittelohrentzündung und Heiserkeit Verwendung bekannt ist.

In weiterer Ausgestaltung der Erfindung ist denkbar, dass - sofern in dem Arzneimittel enthalten - die Bestandteile Cepa, Euphrasia und Mercurius solubilis in der Potenz D12 und Belladonna in der Potenz D15 vorliegen. Grundsätzlich ist es ebenfalls denkbar, die Wirkstoffe in davon abweichenden Potenzen einzusetzen.

Es hat sich als besonders vorteilhaft erwiesen, wenn der Bestandteil Nisylen in dem Arzneimittel in größerer Menge bzw. Konzentration vorliegt als die weiteren Bestandteile.

Vorzugsweise enthält das Arzneimittel Nisylen, Cepa, Euphrasia, Belladonna und Mercurius solubilis, wobei Nisylen in dem Arzneimittel in größerer Menge vorliegt als die weiteren Bestandteile.

Besonders vorteilhaft ist es, wenn der Bestandteil Nisylen in zwei- bis vierfacher, vorzugsweise in dreifacher Menge vorliegt, wie jeder der weiteren Wirkstoffe des Arzneimittels.

Nach einer weiteren Ausgestaltung der Erfindung ist vorgesehen, dass das Arzneimittel Nisylen in einer Menge 4,0 bis 4,5 g, vorzugsweise in einer Menge von 4,286 g, und Cepa, Euphrasia, Belladonna und Mercurius solubilis jeweils in einer Menge von 1,2 bis 1,6 g, vorzugsweise in einer Menge von 1,429 g je 10 ml Lösung enthält.

Es ist denkbar, das Arzneimittel zum Einnehmen und/oder zum Auftragen auszuführen. Letzteres kann beispielsweise bei der Behandlung von Herpes labialis von Relevanz sein.

In bevorzugter Ausgestaltung liegt das Arzneimittel in flüssiger Form vor, wobei es sich um eine alkoholische Lösung zum Einnehmen handeln kann. Auch andere Lösungsmittel als Alkohol sind denkbar. Grundsätzlich ist ebenfalls denkbar, das Arzneimittel in fester Form, beispielsweise als Pulver, Tablette oder als Globuli vorzusehen. Auch ist es möglich, das Arzneimittel in pastöser Form bzw. als Salbe bereitzustellen.

Weitere Einzelheiten und Vorteile der Erfindung werden anhand eines im Folgenden dargestellten Ausführungsbeispiels näher erläutert.

Das Arzneimittel gemäß dem hier beschriebenen Ausführungsbeispiel enthält folgende Bestandteile:

| | |
|---|---|
| Nisylen: | 4,286 g |
| Cepa D12: | 1,429 g |
| Euphrasia D12: | 1,429 g |
| Belladonna D15: | 1,429 g |
| Mercurius solubilis D12: | 1,429 g. |

Diese Bestandteile liegen einer alkoholischen Lösung in einem Volumen von 10 ml vor.

Wie oben ausgeführt, ist eine derartige Lösung in hervorragender Weise zur Behandlung von Herpes genitalis sowie von Herpes labialis geeignet.

Dabei kann vorgesehen sein, dass beispielsweise 3 x 5 Tropfen pro Tag bei Erwachsenen und 3 x 2 Tropfen pro Tag bei Kindern eingenommen werden. Besonders bevorzugt ist eine regelmäßige Einnahme des Arzneimittels.

Denkbar ist ebenfalls, im akuten Stadium oder bevorzugt im Prodromalstadium eine halbstündliche bis stündliche Applikation, das heißt pro halber Stunde bis Stunde z.B. fünf (Erwachsene) bzw. zwei Tropfen (Kinder) der Lösung einzunehmen.

Als vorteilhaft hat es sich erwiesen, die Lösung nicht zu verdünnen und möglichst lange im Mund zu behalten. Als vorteilhaft hat es sich weiter erwiesen, viel zu trinken, möglichst ca. 3 l / Tag.

Wie oben ausgeführt, kommt es bei den oben genannten Herpes-Erkrankungen häufig zu Rezidiven, die ein- oder mehrmals pro Jahr, ggf. auch monatlich auftreten können. Die bisher bekannten Medikamente helfen im akuten Stadium durch Abkürzung des Krankheitsverlaufes und ggf. Linderung der Beschwerden, können jedoch nicht das Auftreten der Rezidive verhindern. Zu dem ist es mittlerweile zu Resistenzen gegen die bekannten Medikamente gekommen.

Die vorliegende Erfindung eröffnet die Möglichkeit, mit einem besonders wirksamen Medikament eine effektive Behandlung von Herpes genitalis sowie von Herpes labialis zu erzielen.

Das Arzneimittel ist als Grippemittel bekannt. Hinweise aus dem Stand der Technik auf die Behandlung der weit verbreiteten Krankheiten Herpes genitalis und Herpes labialis liegen nicht vor.

Grundsätzlich sind auch Abweichungen von dem oben näher beschriebenen Ausführungsbeispiel der Erfindung umfasst. Bei dem Lösungsmittel muss es sich nicht um ein alkoholisches Lösungsmittel handeln. Grundsätzlich kommen auch andere geeignete Lösungsmittel in Betracht. Wie oben ausgeführt, ist die Erfindung weiter nicht auf die Applikation als flüssiges Medikament beschränkt. Ebenfalls ist es denkbar, das Medikament in fester Form (Tablette, Pulver, Globuli) oder als Salbe bereitzustellen.

## Patentansprüche

1. Verwendung eines ersten Arzneimittels mit den Wirkstoffen Aconitum Dil. D3, Gelsemium Dil. D3, Ipecacuanha Dil. D3, Phosphorus Dil. D5, Bryonia Dil. D2 und Eupatorium perfoliatum (Nisylen) zur Herstellung eines Arzneimittels zur präventiven und therapeutischen Behandlung von Herpes labialis und Herpes genitalis.

2. Verwendung nach Anspruch 1, **dadurch gekennzeichnet, dass** das Arzneimittel das erste Arzneimittel mit den Wirkstoffen Aconitum Dil. D3, Gelsemium Dil. D3, Ipecacuanha Dil. D3, Phosphorus Dil. D5, Bryonia Dil. D2 und Eupatorium perfoliatum (Nisylen) als Hauptbestandteil und als weitere Bestandteile Cepa und/oder Euphrasia und/oder Belladonna und/oder Mercurius solubilis enthält, wobei die weiteren Bestandteile als Nebenbestandteile enthalten sind.

3. Verwendung nach einem der vorhergehenden Ansprüche, **dadurch gekennzeichnet, dass** die Bestandteile Cepa, Euphrasia und Mercurius solubilis in der Potenz D12 und Belladonna in der Potenz D15 vorliegen.

4. Verwendung nach einem der vorhergehenden Ansprüche, **dadurch gekennzeichnet, dass** das Arzneimittel das erste Arzneimittel mit den Wirkstoffen Aconitum Dil. D3, Gelsemium Dil. D3, Ipecacuanha Dil. D3, Phosphorus Dil. D5, Bryonia Dil. D2 und Eupatorium perfoliatum (Nisylen), Cepa, Euphrasia, Belladonna und Mercurius solubilis enthält, wobei das erste Arzneimittel mit den Wirkstoffen Aconitum Dil. D3, Gelsemium Dil. D3, Ipecacuanha Dil. D3, Phosphorus Dil. D5, Bryonia Dil. D2 und Eupatorium perfoliatum (Nisylen) in dem Arzneimittel in größerer Menge vorliegt als die weiteren Bestandteile.

5. Verwendung nach einem der vorhergehenden Ansprüche, **dadurch gekennzeichnet, dass** der Bestandteil erstes Arzneimittel mit den Wirkstoffen Aconitum Dil. D3, Gelsemium Dil. D3, Ipecacuanha Dil. D3, Phosphorus Dil. D5, Bryonia Dil. D2 und Eupatorium perfoliatum (Nisylen) in zwei- bis vierfacher, vorzugsweise in dreifacher Menge vorliegt, wie jeder der anderen Bestandteile.

6. Verwendung nach einem der vorhergehenden Ansprüche, **dadurch gekennzeichnet, dass** das Arzneimittel das erste Arzneimittel mit den Wirkstoffen Aconitum Dil. D3, Gelsemium Dil. D3, Ipecacuanha Dil. D3, Phosphorus Dil. D5, Bryonia Dil. D2 und Eupatorium perfoliatum (Nisylen) in einer Menge 4,0 bis 4,5 g, vorzugsweise in einer Menge von 4,286 g, und Cepa, Euphrasia, Belladonna und Mercurius solubilis jeweils in einer Menge von 1,2 bis 1,6 g, vorzugsweise in einer Menge von 1,429 g je 10 ml Lösung enthält.

7. Verwendung nach einem der vorhergehenden Ansprüche, **dadurch gekennzeichnet, dass** das Arzneimittel in flüssiger Form vorliegt.

8. Verwendung nach Anspruch 7, **dadurch gekennzeichnet, dass** es sich bei dem Arzneimittel um eine alkoholische Lösung handelt.

9. Verwendung nach einem der Ansprüche 1 bis 6, **dadurch gekennzeichnet, dass** das Arzneimittel in fester oder pastöser Form vorliegt.

## Claims

1. Application of a first medicinal product with the active constituents Aconitum dil. D3, Gelsemium dil. D3, Ipecacuanha dil. D3, Phosphorus dil. D5, Bryonia dil. D2 and Eupatorium perfoliatum (Nisylen) for the production of a medicinal product for preventative and therapeutic treatment of herpes labialis and herpes genitalis.

2. Application according to Claim 1, **characterised in that** the medicinal product contains the first medicinal product with the active constituents Aconitum dil. D3, Gelsemium dil. D3, Ipecacuanha dil. D3, Phosphorus Dil. D5, Bryonia dil. D2 and Eupatorium perfoliatum (Nisylen) as its main constituents, with additional constituents being Cepa and/or Euphrasia and/or Belladonna and/or Mercurius solubilis, whereby the additional constituents are minor constituents.

3. Application according to one of the preceding claims, **characterised in that** the constituents Cepa, Euphrasia and Mercurius solubilis are present in the potentiation D12, and Belladonna is present in the potentiation D15.

4. Application according to one of the preceding claims, **characterised in that** the medicinal product contains the first medicinal product with the constituents Aconitum dil. D3, Gelsemium dil. D3, Ipecacuanha dil. D3, Phosphorus dil. D5, Bryonia dil. D2, Eupatorium perfoliatum (Nisylen), Cepa, Euphrasia, Belladonna and Mercurius solubilis, whereby there are more constituents of the first medicinal product Aconitum dil. D3, Gelsemium dil. D3, Ipecacuanha dil. D3, Phosphorus dil. D5, Bryonia dil. D2 and Eupatorium perfoliatum (Nisylen) than the other constituents.

5. Application according to one of the preceding claims, **characterised in that** the composition of the first medicinal product is such that the constituents Aconitum dil. D3, Gelsemium dil. D3, Ipecacuanha dil. D3, Phosphorus dil. D5, Bryonia dil. D2 and Eupatorium perfoliatum (Nisylen) are two to four, preferably three, times the amount more than each of the other constituents.

6. Application according to one of the preceding claims, **characterised in that** the medicinal product contains the first medicinal product with the constituents Aconitum dil. D3, Gelsemium dil. D3, Ipecacuanha dil. D3, Phosphorus dil. D5, Bryonia dil. D2 and Eupatorium perfoliatum (Nisylen) in a weight of 4.0g to 4.5g, preferably 4.286g, with Cepa, Euphrasia, Belladonna and Mercurius solubilis each being 1.2 to 1.6g preferably 1.429g, per 10ml.

7. Application according to one of the preceding claims, **characterised in that** the medicinal product is present in liquid form.

8. Application according to Claim 7, **characterised in that** the medicinal product comprises an alcoholic solution.

9. Application according to one of Claims 1 to 6, **characterised in that** the medicine is either in solid or pasty form.

## Revendications

1. Utilisation d'un premier médicament avec les substances actives aconitum dil. D3, gelsemium dil. D3, ipecacuanha dil. D3, phosphorus dil. D5, bryonia dil. D2 et eupatorium perfoliatum (nisyline) pour la fabrication d'un médicament pour le traitement préventif et thérapeutique de l'herpès labial et de l'herpès génital.

2. Utilisation selon la revendication 1, **caractérisée en ce que** le médicament contient le premier médicament avec les substances actives aconitum dil, D3 gelsemium dil. D3, ipecacuanha dil. D3, phosphorus dil. D5, bryonia dil. D2 et eupatorium perfoliatum (nisyline) comme composant principal et comme autres composants cepa et/ou euphrasia et/ou belladona et/ou mercurius solubilis, les autres composants étant des composants secondaires.

3. Utilisation selon l'une des revendications précédentes, **caractérisée en ce que** les composants cepa, euphrasia et mercurius solubilis sont présents à la puissance D12 et la belladonna à la puissance D15.

4. Utilisation selon l'une des revendications précédentes, **caractérisée en ce que** le médicament contient le premier médicament avec les substances actives aconitum dil. D3, gelsemium dil. D3, ipecacuanha dil. D3, phosphorus dil. D5, bryonia dil. D2 et eupatorium perfoliatum (nisyline), cepa, euphrasia, belladonna et mercurius solubilis, le premier médicament avec les substances actives aconitum dil. D3, gelsemium dil. D3, ipecacuanha dil. D3, phosphorus dil. D5, bryonia dil. D2 et eupatorium perfoliatum (nisyline) étant présent dans le médicament en plus grande quantité que les autres composants.

5. Utilisation selon l'une des revendications précédentes, **caractérisée en ce que** le composant le premier médicament avec les substances actives aconitum dil. D3, gelsemium dil. D3, ipecacuanha dil. D3, phosphorus dil. D5, bryonia dil. D2 et eupatorium perfoliatum (nisyline) est présent en quantité double à quadruple, de préférence triple de celle des autres composants.

6. Utilisation selon l'une des revendications précédentes, **caractérisée en ce que** le médicament contient le premier médicament avec les substances actives aconitum dil. D3, gelsémium dil. D3, ipecacuanha dil. D3, phosphorus dil. D5, bryonia dil. D2 et eupatorium perfoliatum (nisyline) en une quantité de 4,0 à 4,5 g, de préférence en une quantité de 4,286 g, et cepa, euphrasia, belladona et mercurius solubilis respectivement en une quantité de 1,2 à 1,6 g, de préférence en une quantité de 1,429g par 10 ml de solution.

7. Utilisation selon l'une des revendications précédentes, **caractérisée en ce que** le médicament est présenté sous forme liquide.

8. Utilisation selon la revendication 7, **caractérisée en ce que** dans le cas du médicament, il s'agit d'une solution alcoolique.

9. Utilisation selon l'une des revendications 1 à 6, **caractérisée en ce que** le médicament est présent sous forme solide ou pâteuse.
